Europäisches Patentamt

(19) European Patent Office

Office européen des brevets

(11) Publication number: **0 191 630 B1**

# (12) EUROPEAN PATENT SPECIFICATION

(45) Date of publication of patent specification :
05.06.91 Bulletin 91/23

(51) Int. Cl.⁵: **A61B 17/22**

(21) Application number: **86300934.6**

(22) Date of filing: **11.02.86**

(54) Catheter with high speed drive means.

(30) Priority: 13.02.85 US 701063
19.06.85 US 746220

(43) Date of publication of application:
20.08.86 Bulletin 86/34

(45) Publication of the grant of the patent:
05.06.91 Bulletin 91/23

(84) Designated Contracting States:
AT BE CH DE FR GB IT LI LU NL SE

(56) References cited:
EP-A- 0 117 519
CH-A- 547 091

(56) References cited:
DE-A- 2 933 266
GB-A- 1 235 321
US-A- 4 020 847
US-A- 4 445 509

(73) Proprietor: KENSEY NASH CORPORATION
Marsh Creek Corporate Center 55East
Uwchlan Avenue Suite 204
Exton, PA 19341 (US)

(72) Inventor: Nash, John
145 Oak Street
Downington Pennsylvania 19335 (US)

(74) Representative: Shaw, Laurence
George House George Road
Edgbaston Birmingham B15 1PG (GB)

Jouve, 18, rue Saint-Denis, 75001 PARIS

## Description

This invention relates to a recanalizing catheter for intravascular or other surgery.

In United States Patent No. 4,445,509 (Auth) there is disclosed a catheter apparatus for recanalizing (opening) a passageway, e.g., an artery, which has been occluded by intra-arterial deposits of atherosclerotic plaque. That recanalization catheter includes a multi-fluted, rotary cutting head mounted at the distal end of the catheter and arranged to be rotated by a flexible drive shaft extending through the catheter and powered by an electric motor at the proximal end thereof. The drive shaft is a steel helical coil approximately 1.3 mm in diameter. Such a coil is stated in the patent to be successful in transmitting high rotational speed (greater than 25,000 rpm) in a controlled fashion and with mechanical security.

The catheter should be long, e.g., 66 to 100 cm or more, while its outside diameter, at least adjacent the working end, should be small, e.g., 3 to 4 mm. The catheter should be able to bend through a minimum diameter radius of curvature of about 7 mm or less, in order to reach small, remotely located restrictions, e.g., occlusions.

The torsional shear stress produced on a flexible drive shaft (e.g., a wire) will differ for different wires, e.g., approximately $10.3 \times 10^8$ Pa for steel wires, 4.8 $\times 10^8$ Pa for beryllium-copper wires. If the radius of curvature through which the drive shaft must bend is very small, e.g., less than 7 mm, high bending stresses will be induced therein. In order to reduce bending strain the diameter of the flexible drive shaft or wire must be made very small, e.g., 0.05 mm or less. If the head is to be operated at a high speed, e.g., greater than 20,000 rpm to provide sufficient power at low torque, the deleterious dynamic effects of critical whirl and friction caused by high side loads in the bearing surfaces supporting the drive wire must be overcome or minimised otherwise the excessive vibration can cause damage. It is one object of this invention to provide a catheter as described and which can be operated at high speed without the risk of undesired side effects.

In one aspect the invention provides a catheter for use in clearing an obstruction in a lumen of a living being, the catheter comprising a flexible tubular member having an end to be located distally of the operator and another end to be located proximally of the operator, a working tool present at the distal end, a rotary drive shaft extending through the tubular member for driving the working tool, power means present at the proximal end of the tubular member for rotating the drive shaft, characterised in that centering means is provided to retain the drive shaft centrally within the tubular member along its length, and the centering means contacts the inner catheter wall and the drive shaft at a plurality of longitudinally spaced apart locations along the length thereof.

In one preferred embodiment, the centering means comprises bearing surfaces which support the drive means at longitudinally spaced locations therealong to maintain the drive means at a longitudinal neutral or central position within the catheter as the catheter is bent through any arc up to the minimum radius of curvature. Preferably the bearings have an opening therein forming a bearing surface through which the drive means extends, the bearings being arranged to pivot with respect ot each other as the catheter is bent so that the drive shaft is maintained in the neutral position.

In a much preferred feature, spacers are present between adjacent bearings to maintain them a predetermined distance apart. Preferably the spacers have a pair of flared free ends to receive ball like bearings. It is preferred that the opening of the bearing is sufficiently short to provide adequate clearance for the drive means when the catheter is bent to the minimum radius of curvature.

Preferably the distal end of the catheter includes a fixed bearing in which connector means is disposed, the connector means being secured to the distal end of the drive means and releasably connected to the tool.

In another preferred embodiment the centering means comprises a spiral of at least one wire wrapped about the other. Preferably the bearing means is formed as a spiral of a wire and the drive means is formed as a spiral of at least one wire in a helix.

In another aspect the invention provides a catheter for use in clearing an obstruction in a lumen in a living being, the catheter comprising an elongate flexible tubular member having an end to be located distally of the operator and an end to be located proximally of the operator, a working tool located at the distal end, rotary drive means extending through the tubular member for driving the tool, and power means located at the proximal end of the tubular member for driving the drive means characterised in that the rotary drive means comprises a pair of interlaced helical wires extending about a fixed guide wire which supports the helical wires along their length, the outer periphery of the coils of the helical wires being spaced slightly from the inner wall of the tubular member, whereby the drive means may be rotated at high speed without excessive vibration about the guide wire, but is also centred within the tubular member.

In order that this invention may be readily understood it will now be described with reference to the accompanying drawings wherein :

Figure 1 is a side elevation partly in section, showing the distal end of one catheter device of the invention ;

Figure 2 is an enlarged sectional view taken along line II-II of Figure 1 ;

Figure 3 is an end view of the catheter ;

Figure 4 is a sectional view taken along line IV-IV on Figure 2 ;

Figure 5 is a sectional view taken along line V-V on Figure 2 ;

Figure 6 is a sectional view taken along line VI-VI on Figure 2 ;

Figure 7 is a sectional view of another catheter ;

Figure 8 is an end view of the catheter of Figure 7 ;

Figure 9 is a sectional view taken along line IX-IX on Figure 7 ;

Figure 10 is a sectional view taken along line X-X on Figure 7 ;

Figure 11 is an enlarged perspective view of one bearing of the drive assembly ;

Figure 12 is a side elevation partly in section showing the distal end of another catheter of this invention ;

Figure 13 is a sectional view taken along line XIII-XIII on Figure 12 ;

Figure 14 is a sectional view taken along line XIV-XIV on Figure 12 ;

Figure 15 is a sectional view taken along line XV-XV on Figure 12 ;

Figure 16 is a side elevation view, partly in section, showing the distal end of another catheter of this invention ;

Figure 17 is a sectional view taken along line XVII-XVII on Figure 16 ;

Figure 18 is a sectional view taken along line XVIII-XVIII of Figure 16 and

Figure 19 is a sectional view taken along line XIX-XIX on Figure 16.

The recanalizing catheter 1 is an elongate, flexible device having a distal end portion 2 at which a working head or tool 3 is present, and a proximal end portion 4, which is adapted to be connected to a rotary power source, e.g., an electric motor (not shown). The catheter includes a drive assembly 5 to rotate the head 3 under the power from the power source.

The working head 3 comprises a rotary cutter 6 having a pair of convex sections 7 which are slightly laterally offset from each other along a divider line 8. The intersection of the convex surface 9 of each section 7 with a planar surface 10 contiguous with the divider line 8 forms an arcuate cutting edge or blade 11. The cutter may be made up of any number of sections, thereby having any number of arcuate blades, with each blade preferably including at least one portion having a negative or zero degree rake. In the embodiment shown each blade is at a negative rake angle of approximately 10°.

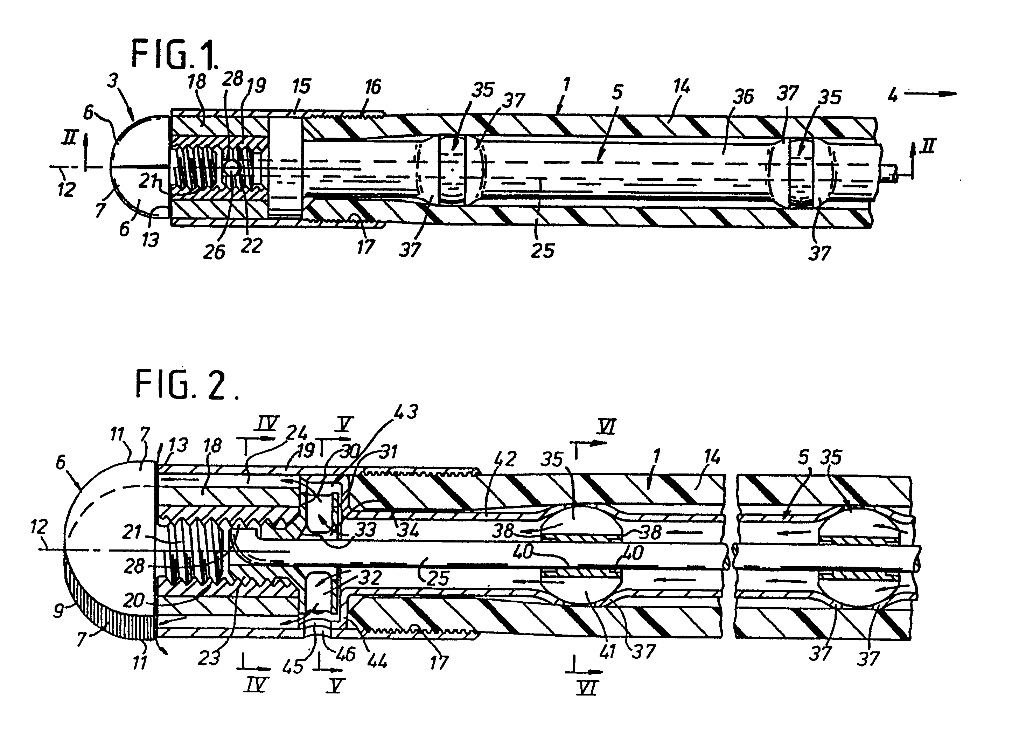

As can be seen in Figure 2 the radial distance of the cutting edge 11 of each blade immediately adjacent the proximal face of the cutter, when measured from the longitudinal central axis 12, is slightly longer than the radial distance from that axis to the outside surface of the distal end portion of the catheter. This feature ensures that a slight space is created between the inner surface of the artery wall and the entrance to the interface 13 between the cutter 6 and the end of the catheter, to prevent any snagging or spooling action of the fibrous tissue of the artery wall within the interface.

The catheter 1 comprises an elongate flexible tubular member 14 having a small outside diameter, e.g., 3 mm (10 French) or less and an inside diameter of approximately 1.8 mm. At the distal end 2 of the catheter a sleeve 15, forming part of the mount for the cutter 6 is threaded by internal threads 16 to threads 17 on the distal end 2 of the catheter. A sleeve 18 is mounted within the sleeve 15 and supports a bearing 19 for rotation about axis 12. The bearing 19 is a cylinder within the bore of the sleeve 18 and serves as a releasable connector for connecting the cutter 6 to the distal end of the drive assembly 5. The bearing 19 includes a threaded bore 20 to engage a threaded short extension 21 projecting from the proximal face of the cutter 6 and on the central axis 12. The distal end of the drive assembly 5 includes drive shaft connector 22 which has an externally threaded, central short extension 23 which is also engaged in the bore 20 of the bearing 19.

As can be seen in Figure 4, the sleeve 18 includes a pair of V-shaped grooves 24 extending longitudinally along the bearing. The grooves 24 are enclosed by the contiguous inner surface of the sleeve 15 to form a pair of diametrical fluid passageways for carrying the fluid from the interior of the catheter 1 to egress at the interface 13, as shown by the arrows in Figure 2.

The drive assembly 5 comprises an elongate flexible drive shaft, which in the embodiment shown in Figures 1 to 6 is a length of a solid wire 25 of very small diameter, e.g. 0.5 mm or less and formed of a suitable high strength flexible material, e.g, steel. The wire 25 extends along the central axis 12 of the catheter. The distal end 26 of the wire 25 extends through a central opening 27 in the connector 22 and is bent perpendicular to be locked in a locking slot 28 so that rotation of the connector 22 about axis 12 causes the connector and wire to rotate together.

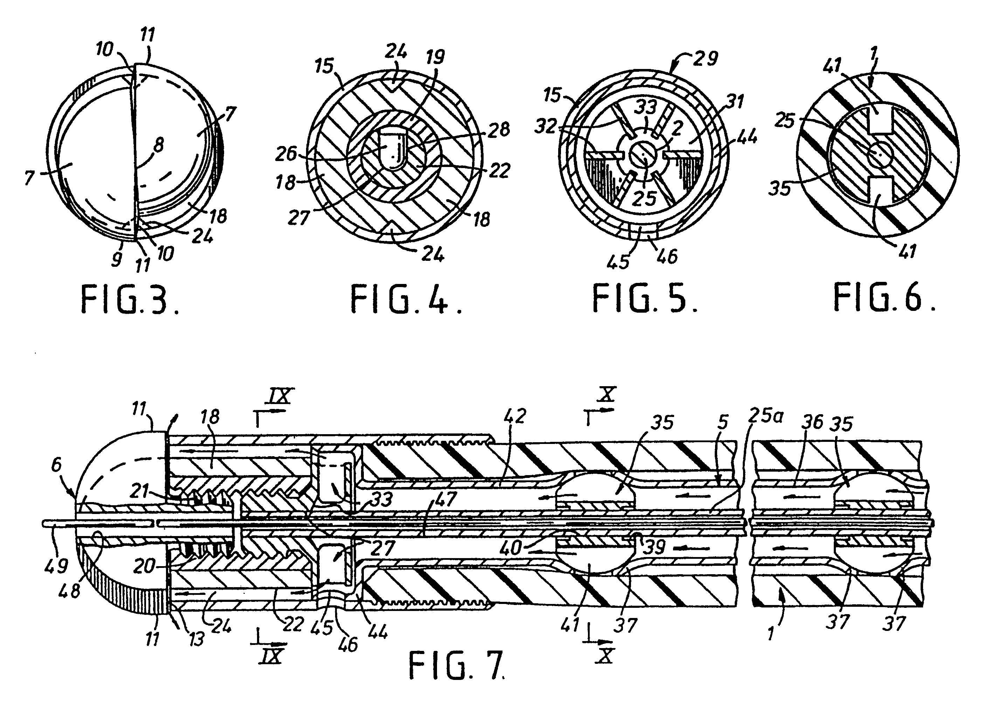

As can be seen in Figures 2 and 5 the proximal end of the connector extension 23 comprises a rotor body 29 having a front wall 30, a rear wall 31, and a plurality of radial spokes or blades 32 projecting outward from a central hub 33 (Figure 5). The central opening 29 in the connector 27 extends through the hub 33 and includes an enlarged bore portion 34 (Figure 2) which is in fluid communication with the spaces between the blades 32.

Fluid to cool and lubricate the drive assembly is arranged to flow through the hollow interior of the catheter 1 past the drive assembly 5 and into the bore 34 from which it flows radially outward and into the proximal ends of the two V-shaped passageways 24, as shown by the arrows in Figure 2.

Spaced apart bearings 35 support the wire 25 longitudinally centrally within the catheter to enable it to rotate at high speed without damage even when the catheter is bent through the very small (minimum) radius of curvature to be expected in intravascular surgical applications, while also precluding the wire from going into critical whirl i.e. excessive vibration as it is rotated at high speed. The bearings 35 are spaced apart by spacer elements 36 so that the centres of the bearings 35, that is the portions supporting the wire 25, are no further apart than one-half of the wavelength of the stationary or standing wave which would result from the rotation of am unsupported wire at the rotational speed. In a preferred embodiment of the invention for wires of no larger than approximately 0.5 mm in diameter and which are to be rotated at speeds in excess of 20,000 rpm, the spacing (pitch) of the bearings should be no larger than 0.64 cm and preferably 0.95 cm.

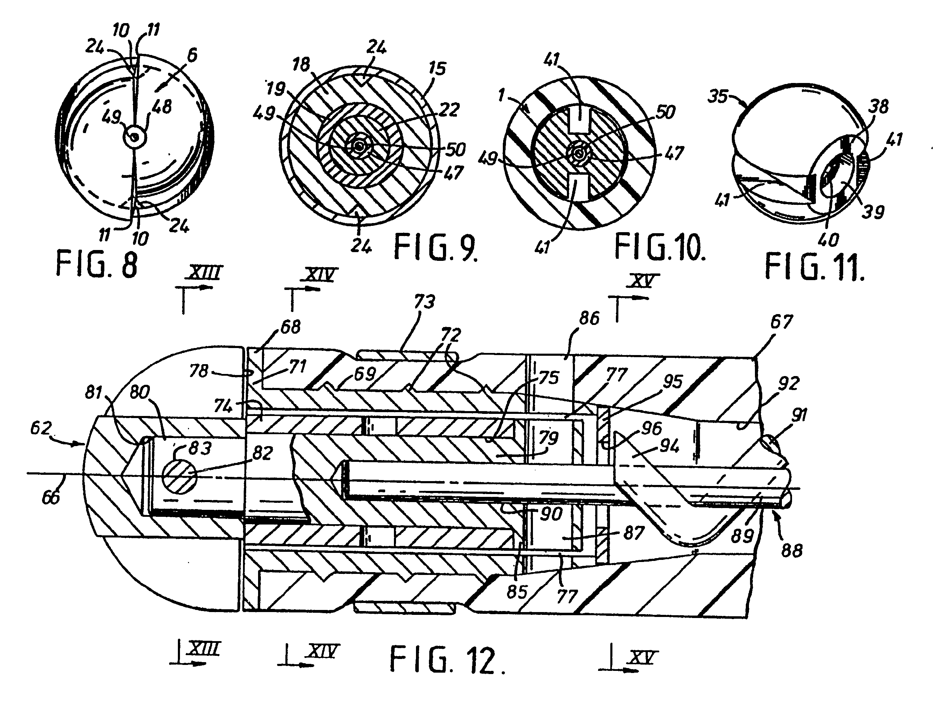

As can be seen clearly in Figures 1 and 2, each spacer 36 is an elongate tube having a pair of flared ends 37. Each bearing 35 is located between the trailing flared end 37 of one spacer and the leading flared end 37 of the adjacent spacer. A typical bearing 35 is shown in perspective in Figure 11, in elevation in Figure 1, and in section in Figure 2. By their shape and disposition the flared ends 37 of spacers 36 form a raceway or seat for the ball like bearings 35. Because ends of adjacent spacers are slightly separated from each other, the spacers can pivot with respect to each other about the intermediate bearing 35 when the catheter is bent, that is, the central longitudinal axis of each of any two immediately adjacent spacers will extend at an angle to the other instead of being colinear as is shown in Figure 2. The greatest deviation from colinearity that adjacent spacers can assume with respect to each other is a function of the geometry of the bearings and spacers, and, in the preferred embodiment disclosed herein, occurs when the catheter is bent through the minimum radius of curvature of 7.5 cm. At this point, edge portions of contiguous spacers abut to preclude further bending of the catheter.

Each of the bearings 35 includes a pair of flat end walls 38 connected by a central passageway 39 and perpendicular to the axis 12. The ends of the passageway 39 are slightly greater in diameter than the intermediate portion to form a bearing surface 40 which has a diameter just slightly larger than the diameter of the wire 25 to support the wire centrally. Each surface 40 forms a flex point for the wire 25 to enable the catheter to be bent while maintaining the wire centrally. When the catheter is bent through an arc the spacers 36 pivot with respect to each other while the wire 25 bends about the pivot surface 40 in each bearing 35 bu ios generally straight and centred with respect to the spacer 36 through which it passes.

Each bearing surface 40 is selected to be sufficiently short so that when the catheter is bent through the minimum radius of curvature, the portions of the bearing contiguous with the ends of the opening 39 do not interfere with the wire 25, while being long enough to provide sufficient surface area to accept the anticipated sideloads at the sliding velocities so that damage to the bearings does not occur. In one preferred embodiment, the length of the bearing surface 40 is no smaller than 1 mm.

Each bearing 35 includes at least two diametrical side slots 41 (Figures 6 and 11). Cooling and lubricating fluid introduced into a spacer 36 flows through the spacer, through the slots 41 and to the next spacer along the length of the catheter.

As can be seen in Figure 2, an element 42 is present between the bearing adjacent the sleeve 18 and the nearest spacer 36 acts as a spacer and a bearing locator. The element 42 includes a tubular portion having a flared proximal end 43. The distal end of the element 42 includes an annular flanged portion 44 within the sleeve 15 and receives and holds the proximal end of the connector 22 and directs fluid from the rotor 29 into the grooves 24.

The flanged portion 44 includes a sidewall opening 45 in alignment with a similar opening 46 in the sleeve 15. The aligned openings form a port for the insertion of a tool to lock or unlock the rotor 29 against rotation. When the connector 22 is locked in position, the cutter 6 can be replaced by unscrewing it from the bearing 19.

In use, the catheter is introduced through an opening in the femoral artery at a point in the groin of the patient remote from the prelocated site of the vascular occlusion or blockage in an artery (e.g., a coronary artery). The catheter is passed via the aorta into the heart and then into the coronary artery until the working head is immediately adjacent the restriction, e.g., a partial occlusion or full occlusion formed by the deposit of atherosclerotic plaque or some other material(s), such as waxy and/or calcified atheroma, thickened and/or ilcerated intima, etc.

The cutter 6 is arranged to be rotated at speed, e.g., in excess of 20,000 rpm, about the longitudinal central axis 12. In use, rotating cutter blades 11 engage occluding material. In the case of hard or calcified deposits, an opening in the restriction is created by the blades cutting away or emulsifying particles of the material making up the restriction. Waxy or soft deposits may be mechanically agitated, beaten or otherwise disturbed by the blades to create an opening without such material(s) actually cut-up or removed from the restriction. In either case an opening permitting the flow of blood through the restriction results.

The fluid to cool and lubricate the drive assembly also provides positive pressure to the wall of the artery contiguous with the cutter, thereby causing the artery wall to move slightly outward radially, away from the cutter, so that no damage to the artery wall by the cutter occurs. The rotating cutter blades impart momentum to the exiting fluid, which action applies further positive pressure to the artery wall, thereby further decreasing the chances of snagging of tissues. The flow of fluid through the interface 13 also precludes any fine fibrous tissue of the artery from gaining ingress into the interface where it could snag or accummulate.

The fluid which is passed down the catheter can, if desired, be oxygenated to eliminate distal ischemia during the restriction opening procedure. Also if desired, nitrates, contrast medium or other drugs can be added to the fluid during the procedure.

In Figure 7 there is shown a catheter in which the drive assembly 5 includes a tubular or hollow wire drive shaft 25a and the cutter 6 includes a central opening 48 to enable a guidewire 49 to be passed therethrough to facilitate the placement of the recanalizing catheter at the desired site. The drive assembly 5 of Figure 7 has bearings 35 and spacers 36 which support a longitudinal central flexible drive shaft 47. The drive shaft wire 25a has a central passageway 50 extending the full length thereof. The introduction of the guidewire can be aided by a fluroscope, and a contrast medium can also be introduced into the artery. The recanalizing catheter of Figure 7 is threaded down the guidewire 49, via the opening 48 in the cutter 6 and the passageway 50 in the drive shaft to the restriction to be opened. The catheter is then operated as described above.

The catheter 61 of Figures 12 to 15 comprises a head 62 having an outer generally convex surface 63 with four recesses 64 therein. The intersection of each of the walls of each recess 64 and the convex surface of the cutter forms an arcuate cutting edge or blade 65. Each arcuate blade 65 includes at least one surface portion having zero or negative rake, with the rake being preferably negative at an angle of approximately 10° to 30°.

The cutter head 62 is arranged to be rotated at a speed, e.g., in excess of 20,000 rpm, about the longitudinal central axis 66 of the catheter under power provided from the remote power source, via the flexible drive assembly 5.

The catheter 61 comprises a flexible tubular member 67 which is formed of a suitable material, e.g., plastic, and which has a small outside diameter preferably approximately 3 mm (10 French) or less, and an inside diameter of approximately 1.5 mm (5 French). At the distal end 68 of the catheter tube 67 there is secured an outer bush 69 which forms part of the mount for the cutter head 62. The outer bush 69 comprises an elongate tubular body portion 70 having an annular flange 71 at the distal end 68. The flange portion 71 forms the distal end 68 of the catheter 61. The tubular body 70 has longitudinally spaced apart gripping teeth 72 and is held in place by a retaining band 73 which tightly encircles the catheter tube 67 to cause the teeth 73 to dig into the interior surface of the catheter tube 67. An inner bush 74 is fixed within the central passageway of the outer bush 69 and is also formed of a tough, wear resistant, low friction material, such as beryllium-copper.

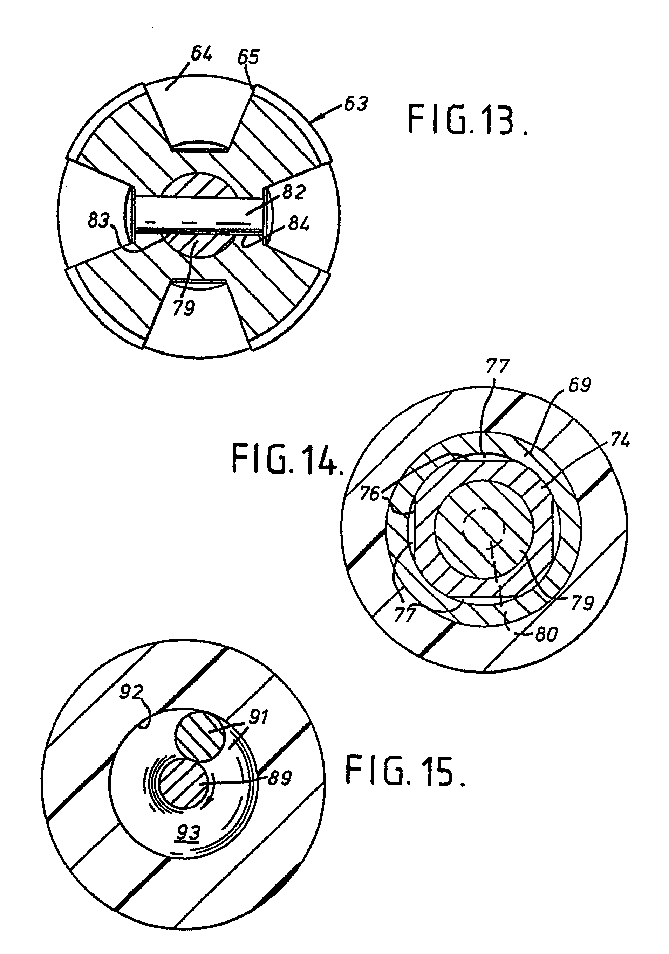

As can be seen in Figures 12 and 14 the inner bush 74 is an elongate sleeve having a central circular passageway 75. The outside wall of the inner bush 74 is generally circular in section but includes four, equadistantly spaced flat portions 76 (Figure 14) which define four spaces 77 between it and the interior surface of the outer bush 69. Each space 77 is in communication with the open interface 78 between the cutting head 62 and the distal end 68 for reasons described below.

A Hollow mounting shaft 79 is located within the central bore 75 in the inner bush 74 and extends along the central axis 66 so that it can freely rotate within the inner bush about that axis. The shaft 79 includes a reduced diameter distal end 80 which is received in within a mating central bore 81 in the cutter head 62 which is secured to the mounting shaft 79 by a retainer pin 82 extending through aligned holes 83 and 84 in the shaft 72 and the cutter head 62, respectively, (Figure 13). The shaft 79 has an annular flange 85 at its proximal end.

A port 86 extends through the catheter tube 67 and an associated port 87 is provided in the flanged portion 85 of the shaft 79. The ports can be aligned to receive a tool to lock the head 62 with respect to the catheter 61.

The catheter 61 contains a flexible drive assembly 88 which comprises an elongate drive wire or shaft 89 which extends the length of the catheter from the power source to the shaft 79. The drive shaft 89 extends into the bore 90 of the shaft 79 and is secured therein.

The drive shaft 89 is arranged to be rotated about the longitudinal axis 66 at high speed to rotate the cutter 62. The assembly 88 includes a bearing 91 to support and maintain the drive shaft 89 at a central (neutral) position within the catheter tube along its length, irrespective of bends in the catheter up to a minimum radius of curvature, e.g., 7.5 cm, while also preventing the drive shaft from going into critical whirl or undue vibration.

The bearing member 91 comprises a helical or spiral cylindrical coil of wire surrounding the flexible drive shaft 89 and is formed of a flexible low friction material such as heat treated beryllium copper.

As can be seen in Figure 15, the outer diameter of the bearing 91 is sufficiently great so that the loops just clear the interior surface 92 of the catheter tube 69 to hold the bearing 91 centered in place. The inside diameter of a central passageway 93 extending the length of the coil bearing 91 is just slightly greater than the outside diameter of the flexible drive shaft 89 so that the drive shaft 89 can rotate freely as shown by the arrow in Figure 15.

As shown in Figure 12, the distal end 94 of the bearing 91 is connected to a stationary disc-like retaining wall 95 which is secured to the proximal end of the outer bush 69. The wall 95 includes a central opening 96 through which the flexible drive shaft 89 extends. The diameter of the opening 96 is sufficiently large to enable liquid introduced into the bore to flow through opening 96 into longitudinally extending passages to egress from the interface 78. The distal end 94 of the helical coil bearing 91 is secured to the wall 95 e.g. by welding.

Because the helical bearing 91 surrounds the drive shaft 89 along its full length while keeping the drive shaft in the longitudinal centre of the catheter the drive shaft cannot go into critical whirl, be subject to undue vibration.

The diameter of the wire making up the helical coil and the pitch of the coil loop and the pitch of the helical coil bearing 91 are selected to ensure that the catheter is sufficiently flexible to negotiate short radia of curvature, while not presenting an undue impediment to the flow of cooling fluid through the passageway 93 in the catheter. In the embodiment shown the diameter of the wire making up the helical coil is approximately 0.25 mm, and the pitch of each coil is approximately 45° which optimizes bending, flexibility, torsional strength and fluid flow passage. The shorter the pitch the more convolutions of the helix and hence the greater distance through which fluid must flow.

As shown the drive shaft is a single wire 89 but instead it can be a rope consisting of a central wire surrounded by six helical wires or a central tube surrounded by helical wires.

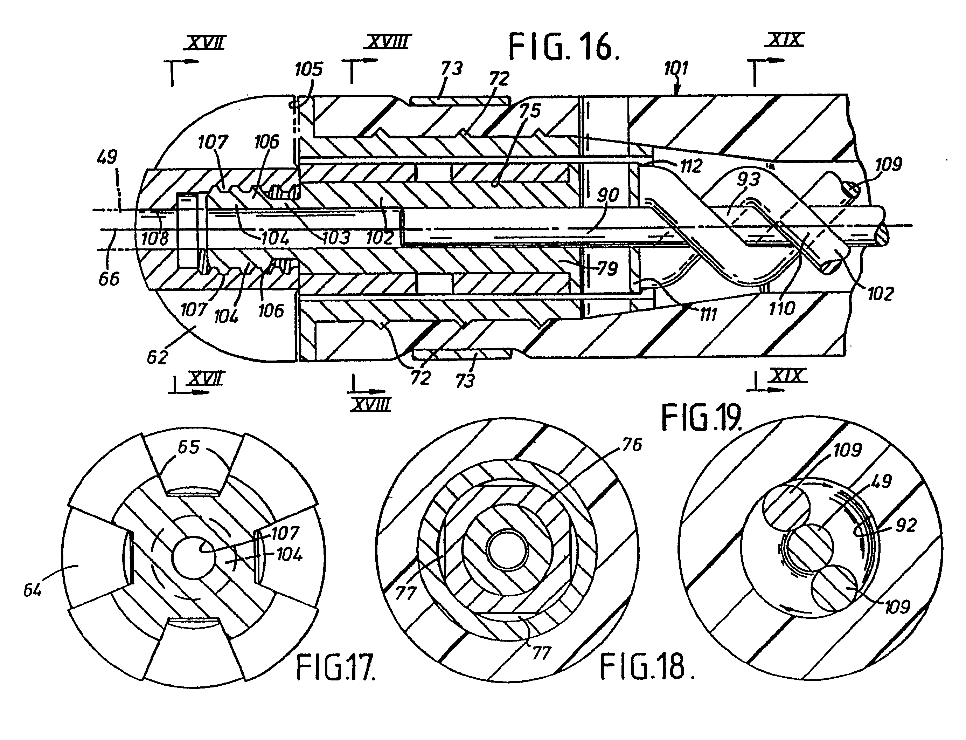

The catheter 101 of Figures 16 to 19 includes a central passageway 93 through which a guide wire 49 can pass to facilitate the placement of the catheter at the desired position. The flexible drive assembly includes an elongated drive shaft, comprising a double helical coil having a longitudinally extending passageway for the guidewire. The head 62 of the catheter 101 is thread mounted, as opposed to being pin mounted like that of the embodiement of Figure 12.

A shaft 102 is present within the central passageway 75 of the inner bush 74. The shaft 102 is a cylinder having a central passageway 103, the distal end 104 of which extends beyond the front face 105 of the outer bush 69. The projecting portion 104 includes peripheral helical threads 106. A matingly threaded bore 107 extends into the cutter 62 for threaded reception of the projecting portion 104 to mount the head 62 on the shaft. A central passageway 108 in the cutter head 62 is aligned with the passageway 103 and a guide wire 49 can be passed.

The flexible drive shaft 5 comprises a pair of interlaced cylindrical helical (spiral) wires 109, 110 which form a passageway 93 about the guide wire 49 for substantially the length of the tube 61. The two helical wires are secured at the distal end to the flanged end 111 of the shaft 102 by suitable means, e.g., weld joints 112.

Each of the wires 109 and 110 forming the flexible drive shaft 5 is of 0.25 mm outside diameter, with the pitch of each coil of the helix being approximately 45°. The inside diameter of the catheter tube and its outside diameter is the same as described with reference to Figure 12.

The outside diameter of the flexible drive shaft 5 is slightly less than the inside diameter of the catheter tube to enable the drive shaft 5 to freely rotate about the central axis.

The outside diameter of the passageway is just slightly greater than the outside diameter of guide wire 49 to enable the two spiral wires 109 and 110 making up the drive shaft 5 to rotate as a unit about the guide wire. The proximal end of the flexible drive shaft 5 is connected by means (not shown) to the electric motor (not shown) to effect such rotation and hence power the catheter 101.

The guide wire 49 is centred in the catheter tube by the passageway 103 at the distal end and by centering means (not shown) at the proximal end thereof. Since the outer periphery of the coils 109, 110 making up the helical drive shaft are spaced slightly from the inner surface of the catheter tube the shaft 26 is enabled to rotate freely about the guide wire, while being centered within the catheter tube even when the tube is bent relatively sharp radia of curvature, e.g. 76 mm. Because the helical drive wires 109 and 110 are supported by the guide wire 49 along substantially the entire length thereof the drive shaft formed by those wires is precluded from going into critical whirl, irrespective of the speed of rotation.

The rotation of the helical drive shaft 5 creates a pumping effect so that the cooling and lubricating liquid introduced into the interior of the tubular catheter at a proximal location is pumped down the length thereof.

The catheter 101 in Figure 16 is guided to the operative position within the artery by the use of the guide wire 49. The catheter is then threaded down the guide wire via the opening 108 and 103 at the distal end thereof and through the central passageway extending down the helical drive shaft 26 until the tip 62 of the catheter is located immediately adjacent the proximal end of the restriction to be opened. As in the other embodiment a suitable liquid is then introduced into the interior of the catheter tube from a point adjacent the proximal end thereof to aid in lubricating and cooling the components within the catheter. The rotation of the helical coil drive shaft causes a pumping action on the liquid to flow down the catheter to the distal end. The liquid flows into communicating passages from whence it flows out of the open interface.

## Claims

1. A catheter (1, 61 Figures 1 to 15) for use in clearing an obstruction in a lumen of a living being, the catheter comprising a flexible tubular member having an end (2) to be located distally of the operator and another end (4) to be located proximally of the operator, a working tool (6, 62) present at the distal end (2), a rotary drive shaft (25, 89) extending through the tubular member for driving the working tool (6, 62), power means present at the proximal end (4) of the tubular member for rotating the drive shaft (25, 89), characterised in that centering means (35, 36, 37, 91) is provided to retain the drive shaft (25, 89) centrally (12) within the tubular member along its length and the centering means (35, 36, 37, 91) contacts the inner catheter wall and the drive shaft (25, 89) at a plurality of longitudinally spaced apart locations along the length thereof.

2. A catheter according to Claim 1 characterised in that the centering means (35, 36, 37 Figures 1 to 11) comprises a plurality of bearings (35) each having an opening (39) therein forming a bearing surface (40) through which the drive shaft (25) extends, the bearings (35) being arranged to pivot with respect to each other as the catheter is bent so that the drive shaft (25) is maintained in the central position.

3. A catheter according to Claim 2 characterised in that the opening (39) of the bearings is sufficiently short to provide adequate clearance for the drive shaft (25) when the catheter is bent to the minimum radius of curvature.

4. A catheter according to Claim 2 or 3 characterised in that spacers (36) are present between adjacent bearings (35) to maintain them a predetermined distance apart.

5. A catheter according to Claim 4 characterised in that the bearings (35) are generally ball-like and the spacers (36) have a pair of flared free ends (37) to receive the ball-like bearings (35).

6. A catheter according to any preceding Claim characterised in that the distal end (2) of the catheter includes a fixed bearing (42) in which connector means (22) is disposed, the connector means (22) being secured to the distal end of the drive shaft (25) and releasably connected to the tool (6, 62).

7. A catheter according to any preceding Claim characterised in that the catheter is adapted so that cooling and lubricating fluid may flow from adjacent the proximal end (4) to the distal end (4) of the catheter through the bearings (35) and out through the interface (13, 78) between the working tool (6, 62) and the distal end of the tubular member (14).

8. A catheter according to Claim 1 characterised in that the centering means (91, Figures 12 to 15) comprises a spiral of at least one wire (91) wrapped about the drive shaft (89).

9. A catheter according to Claim 8 characterised in that the wire (91) is formed of a tough, flexible low friction material such as beryllium copper.

10. A catheter according to Claim 8 or 9 characterised in that the distal end of the wire (91) is secured to a bush (69) at the distal end of the catheter (61).

11. A catheter (101, Figures 16 to 19) for use in clearing an obstruction in a lumen in a living being, the catheter comprising an elongate flexible tubular member having an end (2) to be located distally of the operator and an end (4) to be located proximally of the operator, a working tool (62) located at the distal end (2), rotary drive means (109, 110) extending through the tubular member for driving the tool (62), and power means located at the proximal end (4) of the tubular member for driving the drive means (109, 110) characterised in that the rotary drive means comprises a pair of interlaced helical wires (109, 110) extending about a fixed guide wire (49) which supports the helical wires (109, 110) along their length, the outer periphery of the coils of the helical wires being spaced slightly from the inner wall of the tubular member, whereby the drive means (109, 110) may be rotated at high speed without excessive vibration about the guide wire (49), but is also centred within the tubular member.

12. A catheter according to any preceding Claim characterised in that the tool (3, 62) is releasably connected to the catheter.

## Revendications

1. Cathéter (1, 61 figures de 1 à 15) servant à dégager une obstruction dans un canal naturel chez un être vivant, le cathéter comprenant une partie tubulaire flexible dont une des extrémités (2) est éloignée de l'opérateur et une autre extrémité (4) est proche de l'opérateur, un outil de travail (6, 62) se trouvant disponible à l'extrémité éloignée (2), un arbre rotatif de commande (25, 89) parcourant la longueur de la partie tubulaire pour entraîner l'outil de travail (6, 62), des moyens d'énergie étant présents à l'extrémité (4) la plus rapprochée de la partie tubulaire pour faire pivoter l'arbre de commande (25, 89), caractérisé en ce que des moyens de centrage (35, 36, 37, 91) sont pourvus pour maintenir l'arbre de commande (25, 89) au centre (12) dans la partie tubulaire et dans le sens de la longueur, les moyens de centrage (35, 36, 37, 91) étant en contact avec la paroi intérieure du cathéter et avec l'arbre de commande (25, 89) à plusieurs points espacés sur la longueur de celui-ci.

2. Cathéter qui, selon la revendication 1, est caractérisé en ce que les moyens de centrage (35, 36, 37, figures de 1 à 11) comprennent une série de supports (35) munis chacun d'une ouverture (39) intérieure formant une surface de support (40) au travers de laquelle l'arbre de commande (25) passe, les supports (35) étant arrangés de manière à pivoter l'un par rapport à l'autre quand le cathéter est plié de manière à ce que l'arbre de commande (25) soit maintenu dans une position centrale. .

3. Cathéter qui, selon la revendication 2, est caractérisé en ce que l'ouverture (39) des supports est suffisamment courte que pour fournir un dégagement approprié pour l'arbre de commande (25) quand le cathéter est plié conformément au plus petit rayon de courbe.

4. Cathéter qui, selon les revendications 2 ou 3, est caractérisé en ce que des pièces de séparation (36) sont fixées entre des supports adjacents (35) de manière à les maintenir à une distance prédéterminée l'une de l'autre.

5. Cathéter qui, selon la revendication 4, est caractérisé en ce que les supports (35) ont généralement l'aspect de billes et les pièces de séparation (36) sont munies d'extrémités libres évasées (37) propres à recevoir les supports ressemblant à des billes (35).

6. Cathéter qui, selon toute revendication précédente, est caractérisé en ce que l'extrémité distante (2) du cathéter inclut un support fixé (42) dans lequel des moyens de contact (22) sont disposés, les moyens de contact (22) étant fixés à l'extrémité distante de l'arbre de commande (25) et reliés à l'outil (6, 62) de manière à pouvoir en être détachés.

7. Cathéter qui, selon toute revendication précédente, est caractérisé en ce que le cathéter est adapté de manière à ce que le fluide de refroidissement et de lubrification puisse couler à partir de l'extrémité adjacente (4) vers l'extrémité distante (4) du cathéter en passant par les supports (35) et sortir par l'interface (13, 78) entre l'outil de travail (6, 62) et l'extrémité distante de la partie tubulaire (14).

8. Cathéter qui, selon la revendication 1, est caractérisé en ce que les moyens de cintrage (91, figures de 12 à 15) comprennent au moins un fil métallique en spirale (91) enroulé autour de l'arbre de commande (89).

9. Cathéter qui, selon la revendication 8, est caractérisé en ce que le fil métallique (91) est conçu à partir d'un matériau solide, flexible et à basse friction, tel le cuivre de béryllium.

10. Cathéter qui, selon les revendications 8 ou 9, est caractérisé en ce que l'extrémité distante du fil métallique (91) est fixé à un élément de glissement (69) à l'extrémité distante du cathéter (61).

11. Cathéter (101, figures de 16 à 19) servant à dégager une obstruction dans un canal chez un être vivant, le cathéter comprenant une partie tubulaire allongée flexible dont une des extrémités (2) doit être éloignée de l'opérateur et une autre extrémité (4) près de l'opérateur, un outil de travail (62) étant placé à l'extrémité distante (2), des moyens de commande de rotation (109, 110) se prolongeant au travers de la partie tubulaire pour actionner l'outil (62), et des moyens d'énergie étant situés à l'extrémité (4) proche de la partie tubulaire pour engager les moyens de commande (109, 110), caractérisé en ce que les moyens de commande de rotation comprennent deux fils métalliques hélicoïdaux entrecroisés (109, 110) enroulés autour d'un fil guide fixé (49) qui soutient les fils métalliques hélicoïdaux (109, 110) sur leur longueur, la surface externe des anneaux des fils métalliques hélicoïdaux étant légèrement écartée de la paroi interne de la partie tubulaire, ce qui permet aux moyens d'entraînement (109, 110) de pivoter autour du fil guide (49) à haute vélocité sans trop de vibrations tout en étant centrés dans la partie tubulaire.

12. Cathéter qui, selon toute revendication précédente, est caractérisé en ce que l'outil (3, 62), est relié au cathéter de manière à pouvoir en être détaché.

## Ansprüche

1. Katheter (1, 61, Abb. 1 bis 15) zur Beseitigung eines Hindernisses im Lumen eines Lebewesens, wobei der Katheter aus einem flexiblen, röhrenförmigen Teil besteht, mit einem distal vom Bediener gelegenen Ende (2) und einem proximalen Ende (4), einem am distalen Ende (2) angeordneten Werkzeug (6, 62), einer drehbaren Bedienungswelle (25, 89), die durch den röhrenförmigen Teil zur Bedienung des Werkzeuges (6, 62) führt, mit einem Bedienungselement am proximalen Ende (4) des röhrenförmigen Teils zum Drehen der Bedienungswelle (25, 89), dadurch gekennzeichnet, daß eine Zentriervorrichtung (35, 36, 37, 91) zur zentralisierten (12) Halterung der Bedienungswelle (25, 89) innerhalb des röhrenförmigen Teils vorgesehen ist und die Zentriervorrichtung (35, 36, 37, 91), die innere Katheterwand und Bedienungswelle (25, 89) an mehreren längs angeordnete punkten auf der Länge derselben berührt.

2. Katheter gemäß Anspruch 1, dadurch gekennzeichnet, daß die Zentrierung (35, 36, 37 Abb. 1 bis 11) aus mehreren Lagern (35) besteht, die jeweils eine Öffnung (39) aufweisen, die eine Lagerfläche (40) bildet, durch die die Bedienungswelle (25) verläuft, wobei die Lager so angeordnet sind, daß sie sich bei Krümmung des Katheters so im Verhältnis zueinander drehen, daß die Bedienungswelle (25) stets zentral verläuft.

3. Katheter gemäß Anspruch 2, dadurch gekennzeichnet, daß die Öffnung (39) der Lager kurz genug ist, um der Bedienungswelle (25) bei einem Mindestbiegeradius des Katheters genug Spiel zu gewähren.

4. Katheter gemäß Anspruch 2 oder 3, dadurch gekennzeichnet, daß Distanzstücke (36) zwischen benachbarten Lagern (35) angeordnet sind, um diese in vorbestimmten Abeständen zueinander zu halten.

5. Katheter gemäß Anspruch 4, dadurch gekennzeichnet, daß die Lager (35) normalerweise Kugellager sind und die Distanzstücke (36) zwei aufgeweitete freie Enden (37) zur Aufnahme der Kugellager (35) aufweisen.

6. Katheter gemäß allen vorstehenden Ansprüchen, dadurch gekennzeichnet, daß das distale Ende (2) des Katheters ein festes Lager (42) aufweist, in dem sich eine Steckverbindung (22) befindet, wobei diese Steckverbindung (22) am distalen Ende der Bedienungswelle (25) und lösbar am Werkzeug (6, 62) befestigt ist.

7. Katheter gemäß allen vorstehenden Ansprüchen, dadurch gekennzeichnet, daß der Katheter so angelegt ist, daß Kühlund Schmierflüssigkeit vom proximalen Ende (4) zum distalen Ende (4) des.Katheters, durch die Lager (35) und nach außen durch die Schnittstelle (13, 78) zwischen dem Werkzeug (6, 62) und dem distalen Ende des röhrenförmigen Teils (14) fließen kann.

8. Katheter gemäß Anspruch 1, dadurch gekennzeichnet, daß die Zentriervorrichtung (91, Abb. 12 bis 15) eine Spirale (91) mit mindestens einem Draht enthält, die um die Bedienungswelle (89) verläuft.

9. Katheter gemäß Anspruch 8, dadurch gekennzeichnet, daß der Draht (91) aus einem widerstandsfähigen, biegsamen, reibungsarmen Material wie Berylliumkupfer besteht.

10. Katheter gemäß Anspruch 8 oder 9, dadurch gekennzeichnet, daß das distale Ende des Drahtes (91) in einer Buchse (69) am distalen Ende des Katheters befestigt ist.

11. Katheter (101, Abb. 16 bis 19) zur Beseitigung eines Hindernisses im Lumen eines Lebewesens, wobei der Katheter aus einem länglichen, flexiblen, röhrenförmigen Teil besteht, mit einem distal vom Bediener gelegenen Ende (2) und einem proximal zum Bediener gelegenem (4), einem Werkzeug (62) am distalen Ende (2), einer Bedienungsvorrichtung (109, 110), die durch den röhrenförmigen Teil zur Bedienung des Arbeitswerkzeuges (62) führt und eine Bedienungsvorrichtung am proximalen Ende (4) des röhrenförmigen Teils zur Bedienung der Bedienungsvorrichtung (109, 110), dadurch gekennzeichnet, daß die Bedienungsvorrichtung aus zwei verflochtenen spiralförmigen Drähten (109, 110) besteht, die über einem festen Führungsdraht (49) verlaufen, der die Spiraldrähte (109, 110) auf der ganzen Länge hält, wobei der äußere Umfang der spiralförmigen Drähte die Innenseite des röhrenförmigen Teils nicht berührt, so daß die Bedienungsvorrichtung (109, 110) bei hoher Geschwindigkeit ohne überhöhte Schwingungen des Führungsdrahtes (49) gedreht werden kann, und gleichzeitig auch innerhalb des röhrenförmigen Teiles zentriert wird.

12. Katheter gemäß den vorstehenden Ansprüchen, dadurch gekennzeichnet, daß das Werkzeug (3, 62) lösbar am Katheter angebracht ist.

EP 0 191 630 B1

## FIG.1.

## FIG. 2.

FIG. 3.

FIG. 4.

FIG. 5.

FIG. 6.

FIG. 7.

FIG.11.

FIG.10.

FIG.9.

FIG.8

FIG. 12.

FIG.13.

FIG.14.

FIG.15.

FIG. 16.

FIG.17.

FIG.18.

FIG.19.